# EUROPEAN PATENT APPLICATION

(11) **EP 4 464 352 A1**
(43) Date of publication of application: **20.11.2024**
(21) Application number: 23173329.6
(22) Date of filing: 15.05.2023
(51) Int. Cl.: A61M 11/00, A61M 15/00, A61M 16/00

(54) **INHALATION THERAPY DEVICE**

(71) Applicant: PARI Pharma GmbH, 82319 Starnberg (DE)
(72) Inventor: STOECKL, Carolin, 80331 München (DE); HOFFMANN, Tobias, 86938 Schondorf am Ammersee (DE); HEINE, Benjamin, 80687 München (DE); FILIP, Eric, 81547 München (DE)
(74) Representative: Hoffmann Eitle

(57) **Abstract**

The present disclosure refers to an inhalation therapy device. The inhalation therapy device comprises a housing body (4), a reservoir (2) for holding a liquid, a membrane unit (3), a flow path (7), and a sensor (10). The membrane unit comprises a membrane (5) and an actuator (6). The membrane (5) is disposed in the housing body and has a plurality of apertures, wherein the membrane is disposed so that, when liquid is held in the reservoir, the liquid is supplied to a first side of the membrane. The actuator (6) is coupled to the membrane for vibrating the membrane, whereby the liquid passes through the apertures and an aerosol is generated at a second side of the membrane opposite to the first side of the membrane. The flow path (7) is defined in the housing body and has a first opening (8) to the outside of the housing body at one end and a second opening (9) to the outside of the housing body at another end, so that at least an inhalation flow from the second opening to the first opening is generatable in the flow path upon inhalation of a user at the first opening for entraining and delivering the generated aerosol. The membrane unit (3) is disposed in the flow path (7) between the first opening and the second opening. The sensor (10) comprises a measurement port (11), which is disposed upstream of the membrane unit in the flow path, when seen in the direction of the inhalation flow. The sensor is configured to detect a flow parameter of a flow in the flow path via the measurement port.

## Description

### Technical Field

The present disclosure relates to an inhalation therapy device for entraining and delivering a generated aerosol to a user or patient.

### Background Art

Inhalation therapy devices, also known as (inhalation) nebulisers or aerosol delivery devices, are widely used to deliver therapeutically effective amounts of pharmaceuticals, such as drugs and vaccines, in an aerosol form to users through their respiratory system. Inhalation therapy devices can also be used for diagnostic purposes using radioisotopes for lung challenge tests. For therapy, aerosol inhalation is the preferred root of administration for some pharmaceuticals, which can be intended for treatment of systemic or respiratory diseases.

To achieve the intended treatment, aerosol particles must be deposited in specific parts of the respiratory tract of the user's body, such as the lung. Different size particles tend to deposit in different parts of the respiratory system. It is commonly known in this field that particles having a MMAD (Mass Median Aerodynamic Diameter) of less than Spm or even less than 4µm and at least 1µm are required to allow deposition in the intended part of the respiratory system.

The aerosols for therapeutic purposes are generated and delivered to a desired location within the user's body, especially its respiratory tract, with the inhalation therapy device. To do so, a fluid, particularly a liquid, i.e., a medicament, a drug, a vaccine, etc., to be aerosolised or nebulised is supplied to a reservoir for holding the fluid or liquid in the inhalation therapy device.

For aerosolising or nebulising the liquid in the reservoir, the inhalation therapy devices may, for example, comprise a membrane unit.

Such membrane units comprise a membrane having a plurality of apertures and an actuator that is directly or indirectly, via a support plate supporting the membrane, coupled to the membrane for vibrating the same.

The fluid or liquid may be in contact with a first side of the membrane via gravitational force. The fluid or liquid (i.e., the medicament) passes through the apertures from the first side of the membrane and an aerosol is generated at a second side of the membrane opposite to the first side of the membrane upon vibrating the membrane.

The aerosolised or nebulised fluid or liquid, i.e., the aerosol, inside the inhalation therapy device is then supplied to the user's respiratory system within the bounds of an inhalation therapy upon inhalation of the user through the inhalation therapy device. Meaning, upon the application of a suction force by the user's breathing, especially his or her inhalation, through the inhalation therapy device the aerosol is at least partially transported (by the suction force) from the interior of the inhalation therapy device to the user's respiratory tract.

An example for a conventional inhalation therapy device having such a configuration is, for example, derivable from DE 199 53 317 C1. The membrane unit of the disclosed inhalation therapy device comprises a cylindrical liquid reservoir container, which is delimited at one end face by a membrane having the shape of a circular disc. A liquid disposed in the reservoir contacts the side of the membrane facing the reservoir.

DE 199 53 317 C1 further discloses a membrane that is connected (welded) to a substrate (support plate) and an oscillating generator, for example, a piezo crystal, which surrounds the membrane in a circular manner and is connected (glued) to the substrate, such that the membrane can be caused to oscillate by means of the oscillating generator and an electric drive circuit.

Thereby, the liquid applied to the membrane on the first side of the membrane is conveyed through the apertures in the oscillating membrane to the second side of the membrane opposite to said first side of the membrane to be emitted into a chamber as an aerosol. The disclosed inhalation therapy device further embodies a mouthpiece, or a mask connected to an outlet of the chamber and having an exhalation valve. Upon exhalation of a user, the exhaled flow will, thus, not or only to a limited extend enter the chamber and rather be expelled through the exhalation valve into the environment of the device.

The disclosed device continuously drives the piezo crystal throughout the (single) treatment time (single administration interval), in which substantially the entire liquid in the reservoir is aerosolised by the membrane. During exhalation of a user, the generated aerosol is temporarily stored in the chamber forming an aerosol cloud (bolus), which upon inhalation is inhaled and transported to the user's respiratory tract for therapy. Therefore, the treatment time can be kept relatively short.

One potential drawback is, however, that with an increasing output rate of the aerosol generator (membrane, piezo crystal, drive circuit, etc.) the amount of aerosol deposited on the inner walls of the device (known as a so-called "rainout"), particularly in the chamber, increases. This leads to an increased loss of administrable aerosol/pharmaceutical.

Other conventionally known inhalation therapy devices drive the piezo crystal only during inhalation and stop driving the piezo crystal during exhalation. Those inhalation therapy devices are often referred to as breath-triggered devices or breath-actuated devices. It has been found that such inhalation therapy devices often have the issue of a long therapy duration (long administration interval), which can be annoying to the user and, thereby, provoke a lack of user adherence, e.g., an interruption or a premature end of the therapy before the intended dose of fluid or liquid in the reservoir has been nebulised and delivered to the user's respiratory tract for therapy.

Both of the conventional inhalation therapy devices described above are oftentimes operated in such a manner that the user merely inhales through the inhalation therapy device (an inhalation flow is generated through a flow path of the device), and the inhalation therapy device is removed from the user's mouth and/or nose upon exhalation. While both enables a resistance-free exhalation of the user, the overall treatment cannot be satisfactorily monitored, as no data, such as a flow parameter in a flow path of the device, can be obtained during the exhalation. Additionally, and in case of a continuous operation, generated aerosol gets lost without any therapeutic effect for the user.

On top of that, removing the inhalation therapy device with every exhalation negatively affects the user's "natural" respiration, i.e., his or her steady breathing cycle.

Hence, conventional systems were confronted with a need for an effective inhalation therapy device that enables a short overall therapy time (single administration interval), while rainout and/or loss of generated aerosol without any therapeutic effect for the user can be reduced.

To achieve the same, various control mechanisms for the aerosol generation inside the inhalation therapy device have been proposed. While activating (during inhalation) and deactivating (during exhalation) the driving of the piezo crystal - in general - ensures that losses of the aerosolised fluid or liquid containing a pharmaceutical can be reduced, turning the aerosol generation on and off, i.e., performing the triggering at the right time is rather difficult.

On the one hand, the aerosol generation must start at the latest upon the start of the user's inhalation to omit an unnecessary prolongation of the treatment time. On the other hand, the production of aerosol must be stopped early enough that, upon exhalation of the user through the inhalation device, it can be avoided that produced aerosol is expelled from the inhalation therapy device and the user's upper respiratory tract without any therapeutic application.

To achieve the same, the so-called "pre-on/pre-off" inhalation therapy devices have been considered by the Applicant. Therein, the aerosol production is started in advance of the actual inhalation of the user, such that a certain amount of aerosol is generated before the actual inhalation (i.e., a pre-inhalation aerosol production similar to the aerosol bolus discussed above is produced), and the drive of the piezo crystal is stopped before the exhalation starts, i.e., a pre-exhalation aerosol production stop is performed.

To achieve the same, all non-continuously operating inhalation therapy devices require a precise monitoring of the user's breathing pattern at least in the inhalation direction to adequately control the drive of the piezo crystal and, thereby, the aerosol production.

Accordingly, most conventional non-continuously operating inhalation therapy devices comprise sensors.

The previous solutions are, however, confronted with a deterioration of the quality of the sensor signal during a single or several consecutive administration interval(s). This is due to a contamination of the sensor port, for example, by germs, bacteria, humidity, fluids, and/or sputum stemming, for example, from the exhalation of the user into the inhalation therapy device. This provokes an adulteration of the sensor measurement results, consequently, a malfunction of the inhalation therapy device, and, ultimately, a decreased success of therapy as well as an increased risk of microbiological contamination of the inhalation therapy device.

### Summary of the Disclosure

In view of the aforesaid, it is an object of the present disclosure to provide an inhalation therapy device that increases the overall quality of drug delivery in a consistent and reliable manner.

This object is solved by means of an inhalation therapy device according to independent claim 1. Distinct embodiments are derivable from the dependent claims.

According to the first aspect of the present disclosure, an inhalation therapy device comprises a housing body, a reservoir for holding a liquid, a membrane unit, a flow path, and a sensor.

The membrane unit is arranged inside the housing body and comprises a membrane, which is disposed in the housing body in such a manner that, when a fluid or liquid (i.e., a medicament) to be aerosolised or nebulised is held in the reservoir of the inhalation therapy device, the liquid is supplied to a first side of the membrane.

The membrane unit also comprises an actuator, which is coupled directly or indirectly via a support plate supporting the membrane, to the membrane for vibrating the same. To generate the aerosol, the membrane comprises a plurality of apertures, wherein, upon vibrating of the membrane, the liquid passes through said apertures and an aerosol is generated at a second side of the membrane, which is opposite to the first side of the membrane.

The flow path itself is defined in the housing body and comprises a first opening to the outside (environment) of the housing body (inhalation therapy device) at one end of the flow path and a second opening to the outside of the housing body at another end.

The membrane unit is disposed in the flow path between said first and second openings. This configuration is, however, not limited to such an inline arrangement of the membrane unit in the flow path itself. For example, the membrane unit can also be arranged in a separate channel being in fluid communication with the flow path, such that the generated aerosol can equally be transmitted to the flow path. An example for such a configuration is a T-connection inside the flow path. At the same time, it is possible to arrange the membrane unit in such a manner directly/inline in the flow path that it can at least be partially surrounded by the flow in the flow path. In other words, an envelope flow is generated about the circumference or part of the circumference of the membrane.

A flow in the flow path is, hence, generatable in at least a first flow direction from the second opening to the first opening upon inhalation of the user at the first opening for entraining and delivering the aerosol generated at the second side of the membrane inside the flow path to a user.

The "first opening" may, for example, be understood as a "mouthpiece", an inhalation mask, or the like, or at least a connection to such elements through which the user can apply a suction force to the flow path of the inhalation therapy device.

Accordingly, to establish said flow through the flow path in the first flow direction, i.e., the inhalation flow direction, air must accordingly be able to enter the flow path at the second opening. Accordingly, the "second opening" at least acts as an "entrance" for air when an inhalation of the user at the first opening of the therapy device is performed to entrain and deliver the generated aerosol inside the inhalation therapy device. During an inhalation phase, the second opening may, thus, also be referred to as "inlet opening" and the first opening may, thus, also be referred to as "outlet opening".

The first flow direction may, in view of the above, be interpreted as an "inhalation direction" or "inhalation flow direction" of the (air, preferably air and aerosol) flow through the inhalation therapy device.

It may equally be possible to generate a flow in the flow path in an opposite, second flow direction from the first opening to the second opening upon exhalation of the user at the first opening for expelling the air from the respiratory tract. The second flow direction may, thus, be interpreted as an "exhalation direction" or "exhalation flow direction" of the (air) flow through the inhalation therapy device. During an exhalation phase, the second opening may, thus, also be referred to as "outlet opening" and the first opening may, thus, also be referred to as "inlet opening".

This enables that the inhalation therapy device can stay in contact with the user (for example, his or her mouth, and thereby his or her respiratory tract) during the whole therapy duration (or treatment time) of a single administration interval and the inhalation and the exhalation can be performed through the flow path and the air flow through the inhalation therapy device.

The sensor comprises a measurement port, which is disposed upstream of the membrane unit in the flow path, when seen in the inhalation flow direction, i.e., the first flow direction. The sensor is configured to detect a flow parameter of the flow in the flow path via the measurement port.

When seen in the first flow direction, meaning the inhalation flow direction, the measurement port is arranged "behind" the membrane unit, which thereby acts, at least partially, as a "germ/sputum shield" for the measurement port provided to allow the sensor's detection of a flow parameter of the flow in the flow path. On top of that, it is to be ensured that the measurement port is arranged in a comparably "dry" region inside the inhalation therapy device's flow path, which is beneficial for the quality of detection.

The "flow parameter of the flow in the flow path" may be a parameter regarding the inhalation flow and/or the exhalation flow and, thus, a parameter that allows to monitor the user's breathing pattern during therapy. Accordingly, it is, for example, possible to evaluate one or more of the user's tidal volume, the flow volume, the breathing frequency, the inhalation/exhalation ratio, etc. throughout the entire therapy time.

In a preferred embodiment, the sensor may be a differential pressure sensor, which, on the one hand, measures the pressure, more preferably the static pressure, inside the flow path via the measurement port, and, on the other hand, measures a pressure in the environment (meaning on the outside of the inhalation therapy device) as a reference pressure to determine the pressure values inside the inhalation therapy device during inhalation (and/or exhalation).

Having a measurement port upstream of the membrane unit enables to reliably and precisely detect a flow parameter of the (inhalation and/or exhalation) flow in the flow path. This is particularly the case, as in the area of the flow path upstream of the membrane unit, there is hardly any aerosol (for example, in a non-continuously operating inhalation therapy device), which could, potentially, carry humidity, germs, and/or sputum that could potentially be deposited at the measurement port and, thereby, reduce a measurement accuracy by at least covering the sensor port. Hence, the position of the measurement port enables that a pollution of the measurement port by germ, sputum load, and/or other liquids can be omitted. Hence, the therapy success can be improved due to a more reliable and precise detection of the flow parameter over a single or several consecutive administration interval(s).

Furthermore, providing a sensor with a sensor port, i.e., the measurement port, allows for an arrangement of the sensor away from the flow path. Meaning, the sensor can be arranged in a distanced manner from the actual flow in the flow path and the flow parameter is detected via its measurement port. In other words, the sensor does not have to be arranged directly in the flow path. To the contrary, it can be arranged away, for example, in a cleaner and/or more protected surrounding, such as inside the housing body. The flow parameter detection/measurement is performed via the sensor's measurement port, which is arranged in the flow path.

This is particularly beneficial when, for example, the inhalation therapy device comprises an additional holding portion for holding the inhalation therapy device, and the sensor is arranged therein, while the measurement port is arranged in the inhalation therapy device, particularly in the flow path itself. This configuration helps to reduce the hygienic requirements of the holding portion in terms of disinfection, while the flow parameter detection inside the flow path can still be achieved. This also allows that the inhalation therapy device without the holding portion may, for example, be configured as a cost-efficient throwaway product, a single use product, or a product that is less often used compared to the holding portion comprising the sensor.

To achieve said arrangement, it is required to establish a connection between the sensor and its measurement port. This can, for example, be achieved by a hose, an injection moulded part, or the like.

According to a second aspect, the sensor is configured to at least detect a pressure of the inhalation flow in the flow path. In other words, the sensor in the flow path may be a pressure sensor comprising the measurement port.

It is preferred that the sensor is not only configured to detect, but also to measure, i.e., to continuously detect, a pressure of the inhalation flow in the flow path.

Irrespective thereof, it is preferred that the sensor does not only detect/measure the pressure of the inhalation flow in the flow path, but also detects/measures the pressure of the exhalation flow from the first opening to the second opening in the flow path upon exhalation of the user at the first opening into the flow path.

Nonetheless, the present application is not limited thereto. The sensor may also be a flow sensor, i.e., a sensor for detecting a flow velocity in the flow path, at least in the inhalation flow direction, preferably in the inhalation flow direction and the exhalation flow direction. Equally, the sensor may be a mass flow sensor, a volume flow rate sensor, a pneumotachograph, a differential pressure sensor, or a hot-wire anemometer.

According to a third aspect, the membrane unit is arranged in such a manner in the flow path that it is at least partially, preferably fully surrounded by the flow in the flow path.

In other words, an envelope flow is generatable about the circumference or part of the circumference of the membrane. For example, the membrane unit may be arranged in an axially and circumferentially centred position of the flow path, such that a sheeting and enveloping flow can pass the membrane unit inside the flow path at least during inhalation, and preferably during exhalation of the user through the inhalation therapy device during therapy. This allows a compact and simple structure of the inhalation therapy device, which is easy to clean, disinfect, and manufacture. At the same time, the generated aerosol at the second side of the membrane can easily be entrained and delivered to the user's body. This configuration also advantageously enables a reduced flow rate dependency of the aerosol deposition, as the aerosol is produced inside the flow path at a position that is distanced, i.e., (far) away, from inner walls of the flow path.

According to a fourth aspect, the entire inhalation flow passes through the flow path upon inhalation of the user. That is, the flow path is shaped in such a manner that the entire inhalation flow passes through the flow path upon inhalation of the user. In other words, one single flow path for inhalation through the inhalation therapy device, preferably for inhalation and exhalation through the inhalation therapy device is provided.

This allows that the measurement of the flow parameter, preferably the pressure of the inhalation flow, more preferably the pressure of the inhalation and exhalation flow in the flow path, can be performed in the flow path. Vice versa, it is possible to omit the urge for having separate flow paths, in which a sensor or at least a measurement port thereof is arranged.

In this connection, the "entire inhalation flow" is to be understood as the flow necessary to fill the tidal volume, which is breathed on by the user upon inhalation through the inhalation therapy device and is accordingly moved into the inhalation therapy device and out of it into the user's respiratory tract.

According to a fifth aspect, the measurement port, which is disposed upstream of the membrane unit in the flow path, when seen in the inhalation flow direction, is disposed substantially in the middle between the measurement port and the second opening in the flow path.

Accordingly, an improved quality of the flow parameter detection/measurement can be achieved, as potentially negatively affecting fluid dynamic effects stemming, for example, from walls of the flow path can be omitted.

Alternatively, it may also be possible to arrange the measurement port closer to the second opening than to the membrane unit in the flow path. Preferably, the measurement port is arranged closer to the second opening than to the membrane unit in the flow path and is arranged distanced from the second opening and the membrane unit.

In a further, alternative configuration, the measurement port may be disposed closer to the membrane unit in the flow path than to the second opening.

In an even further configuration, the measurement port may be disposed closer to the membrane unit in the flow path than to the second opening and is disposed distanced from the second opening and the membrane unit.

Any of these configurations enables that the measurement port is arranged in an area that is offset from the main air flow through the inhalation therapy device in the inhalation flow direction, and preferably the exhalation flow direction. Particularly during inhalation, this enables to reliably improve the quality of detecting/measuring the flow parameter, as dynamic effects of the flow path that would deteriorate the detection/measurement can be reduced at the measurement port. At the same time, an arrangement closer to the second opening than to the membrane unit in the flow path allows to further enhance the protection from pollution via aerosol, germs, bacteria, and/or sputum that may distort the measurement of the flow parameter.

According to a sixth aspect, the measurement port comprises a measurement surface. The measurement surface is inclined to the longitudinal axis of the first opening.

The term "measurement surface" may be understood as the area of the measurement port, where the flow parameter, such as a velocity or pressure, is detected. In other words, the measurement surface may be an integral part of said measurement port being arranged in the flow path of the inhalation therapy device. Furthermore, the "longitudinal axis of the first opening" is, in the present context, to be understood as a longitudinal axis that is passing through the end of the inhalation therapy device's flow path, at which the inhalation of the user is performed. In other words, the longitudinal axis is the axis that runs along the end of the flow path at the first opening. Therefore, potentially inclined end faces of the first opening do not affect the orientation of the longitudinal axis of the first opening.

This may, alternatively, also be understood as an inclined orientation of the measurement surface to the horizontal, when the inhalation therapy device is held in an (ideal) position by the user during therapy in a (standing or) sitting position of the user and an upright position of the user's head.

The "ideal position" in this connection is to be understood as an orientation of the inhalation therapy device, in which a contact of the liquid with the first side of the membrane can be ensured. This is, for example, particularly given, when the membrane is arranged substantially vertically and/or the longitudinal axis of the first opening extends horizontally. The inclinations defined above, i.e., the tilting of the measurement surface to the longitudinal axis of the first opening or the inclination of the measurement surface (plane) relative to the horizontal when the therapy device is held by a user in the ideal position as described in the preceding paragraphs, may be in a range of 5° to 80°, preferably 10° to 60°, more preferably, 20° to 50°.

This tilting of the measurement surface during therapy reduces the risk of sputum pollution, pollution via other liquids (such as deposited aerosol), and/or germ pollution on the measurement surface of the inhalation therapy device and, thereby, reliably improves the quality of detecting/measuring the flow parameter in the flow path.

According to a seventh aspect, the measurement port is arranged at a position other than a lowermost position of the flow path, when the inhalation therapy device is held by the user, preferably substantially in an ideal position, during therapy in a standing or sitting position of the user and the head of the user is in the upright position.

Preferably, the measurement port is arranged at a position that is at least 3mm, preferably 5mm, more preferably 7mm, most preferably 10mm away from the lowermost position of the flow path, when the inhalation therapy device is held by the user, preferably the ideal position, during therapy in the standing or sitting position of the user.

It is apparent that an arrangement of the measurement port away from the lowermost position can effectively prevent during therapy that the measurement surface is in contact with any liquid and/or remnants that potentially gathers inside the flow path at the lowermost position due to gravitational force.

According to an eighth aspect, a protruding portion protruding into the flow path is arranged inside the flow path. The measurement port is arranged on said protruding portion.

This protruding portion may, therefore, also be understood as a radially inwardly protruding platform, protest, pedestal, or podium inside the flow path, such that the measurement is arranged in a distanced manner from one of the walls of the flow path.

As such, not only the contamination by sputum or germs can be avoided, but also the quality of the flow parameter detection/measurement can be improved.

It is, according to a nineth aspect, preferred that the protruding portion protrudes into the flow path by at least 0.5mm, preferably by at least 1mm, more preferably by at least 1.2mm, most preferably by at least 1.5mm.

This configuration enhances the effect that not only the contamination by sputum or germs can be avoided, but also the quality of the flow parameter detection/measurement can be improved.

According to a tenth aspect, the flow path is shaped in such a manner that a centre point of the cross-section of the first opening, a centre point of the cross-section of the membrane, and a centre point of the cross-section of the second opening do not align with each other.

This allows for an arrangement that is not considerable as a "tube"-like inhalation therapy device, in which large close-to-wall velocities negatively influence the detection/ measurement of the flow parameter, preferably the pressure, inside the flow path. These negative influences particularly occur in such "tube"-like inhalation therapy devices due to pressure fluctuations, which are commonly caused by separations and instabilities at/close to the inner walls of the flow path and can be effectively avoided by the above-mentioned configuration.

To the contrary, the non-matching orientation of the three centre points of the cross-section of the first and second openings and the membrane requires a detour/redirection of the air flow through the inhalation therapy flow path, which positively affects the flow parameter detection/measurement inside the flow path, as the dynamic share of the to be detected/measured flow parameter, such as pressure values at the measurement port can be reduced. Further, when a volume flow or mass flow is to be detected/measured inside the flow path, the dynamic share can be kept in a range.

According to an eleventh aspect, the second opening is shaped in such a manner that the inhalation flow is at least partially guided towards an inner wall of the flow path.

This allows to reduce large close-to-wall velocities in the relevant areas for the detection/measurement and provokes that an air flow entering the flow path via the second opening upon inhalation needs to be redirected in its path through the flow path.

This allows to arrange the measurement port in an area, in which the flow does not directly contact the measurement port upon inhalation of the user at the first opening for entraining and delivering the generated aerosol from the second side of the membrane.

According to a twelfth aspect, the measurement port of the inhalation therapy device according to the eleventh aspect is arranged away, preferably at least 5mm away from an area, where the inhalation flow contacts the inner wall of the flow path.

This allows to arrange the measurement port in an area, in which the flow does not directly contact the measurement port upon inhalation of the user at the first opening for entraining and delivering the generated aerosol from the second side of the membrane. Put differently, the measurement port is arranged offset from the main air flow through the inhalation therapy device's flow path. This ensures dynamic effects during the detection/measurement of the flow parameter, preferably the pressure, inside the flow path upon inhalation can be kept as low as possible. This advantageously improves the quality of the detected/measured flow parameter signals.

It is to be noted that it is impossible that the entire, meaning 100%, of the inhalation flow contacts the inner wall of the flow path, as the entry of air into the flow path via the second opening upon inhalation at the first opening, causes unavoidable turbulences and flow deviations. Nonetheless, the term "where the inhalation flow contacts the inner wall of the flow path" is to be understood as the area, in which the majority of the inhalation flow entering the flow path via the second opening up on the user's application of suction force at the first opening for entraining and delivering the generated aerosol contacts the inner wall.

According to a thirteenth aspect, the measurement port is arranged offset from a vertical projection of the cross-section of the second opening.

In addition or alternatively to the aforesaid, the measurement port may be arranged offset from a horizontal projection of the cross-section of the second opening.

The second opening acts as an "entrance" of the air into the flow path, upon inhalation of the user through the inhalation therapy device. In this connection, the orientation and arrangement of the measurement port as described above, ensures that the measurement port is arranged away, i.e., is offset, from the main flow through the inhalation therapy device, and thereby, for example, the share of dynamic pressures during the pressure measurement can be reduced.

According to a fourteenth aspect, the measurement port is arranged in an area of the flow path, in which a dynamic pressure is between 0.0001% and 40% of the maximum dynamic pressure inside the flow path. The dynamic pressure is to be measured during a sinus-shaped breathing pattern of two seconds of inhalation time and an exhalation time, respectively, and 500ml of tidal volume.

It is preferred that the measurement port is arranged in an area of the flow path, in which the dynamic pressure is between 0.0005% and 20% of the maximum dynamic pressure inside the flow path when being measured as explained above.

It is more preferred that the measurement port is arranged in an area of the flow path, in which the dynamic pressure is between 0.001% and 5% of the maximum dynamic pressure inside the flow path when being measured as explained above.

It is most preferred that the measurement port is arranged in an area of the flow path, in which the dynamic pressure is between 0.002% and 2% of the maximum dynamic pressure inside the flow path when being measured as explained above.

The lower boundaries for the measurement port arrangement, respectively ensure that the inhalation therapy device's measurement port is in a region of a small to moderate (but existent) air flow. This helps drying off potentially present humidity. This means that the measurement port is arranged in an area, in which a small amount of dynamic pressure is given, which enables that contamination can be transported away from the measurement port. Equally, liquids or the like can be prevented from adhering to the measurement port, while precise flow parameter detection/measurement can be performed during therapy.

Equally, the respective upper boundary for the measurement port arrangement above ensures that the measurement port is arranged away from a section, in which a large flow velocity of the (air) flow through the inhalation therapy device negatively affects the measurement/detection of the pressure values inside the flow path.

Measuring a "peak air flow" of a breathing pattern and/or a "dynamic pressure" thereof, including a "maximum dynamic pressure" is the standardised way of evaluating air flows of anaesthetic and respiratory equipment, such as nebulising systems and components thereof. To do so, it is common practice to apply the above-mentioned sinus-shaped breathing pattern of two seconds of inhalation time, two seconds of exhalation time, and 500ml of tidal volume at anaesthetic and respiratory equipment to establish comparative information between such anaesthetic and respiratory equipment. The details of how these peak air flow values are to be detected/measured, the apparatuses required to do so, as well as the experimental conditions are, for example, reflected in the "DIN standards committee rescue services and hospital" standard DIN ISO 27427.

This international standard was developed to cover "general purpose" inhalation therapy devices and is based on adult test parameters for evaluating the suitability of respective inhalation therapy devices' intended used as disclosed by their manufacturers and to evaluate their safety as well as their compatibility between the materials of the components of the inhalation therapy devices and the dispensed liquids.

Based on this standard DIN ISO 27427, it is justified to consider the above-mentioned "peak flow" or "maximum dynamic pressure" during the application of a standard (experimental) sinus-shaped breathing pattern of two seconds of inhalation and exhalation time, respectively, and a tidal volume of 500ml to the inhalation therapy device, as the maximum pressure values being the absolute pressure values, detected inside the inhalation therapy device during said application of said sinus-shaped breathing pattern. In such a setup, the maximum dynamic pressure is present at the peak flow. Equally, the absolute pressures reach a maximum as well.

It is, therefore, apparent that measuring a "dynamic pressure" of such a breathing pattern is one of many standardised ways of evaluating and observing air flows of anaesthetic and respiratory equipment.

Accordingly, this peak flow corresponds to a flow rate of 23.56 l/min through the inhalation therapy device. The standard setup using a differential pressure sensor with one sensor port inside the device (measuring static pressure) and one sensor port in the environment (measuring reference pressure) may be used to determine the pressure values inside the inhalation therapy device upon application of the above-mentioned breathing pattern.

According to a fifteenth aspect, the measurement port is arranged in an area of the flow path, in which the ratio of the relative static pressure at the measurement port to the relative static pressure at the first opening is between 80% and 120%.

The term "relative static pressure" is to be understood as a difference between a local static pressure and an ambient pressure of the inhalation therapy device.

Also said relative static pressures are to be measured during the well-known above-discussed measurement setup, namely with a measurement during a sinus-shaped breathing pattern of two seconds of inhalation time and exhalation time, respectively, and 500ml of tidal volume.

It is preferred that the above-discussed ratio of the relative static pressure at the measurement port to the relative static pressure at the first opening is between 90% and 110%.

It is even more preferred that the above-discussed ratio of the relative static pressure at the measurement port to the relative static pressure at the first opening is between 95% and 105%.

The lower boundaries for the measurement port arrangement, respectively ensure that the inhalation therapy device's measurement port is in a region of a small to moderate (but existent) air flow. This helps drying off potentially present humidity. Further, liquids or the like can be prevented from adhering to the measurement port, while precise flow parameter detection/measurement can be performed during therapy.

Equally, the respective upper boundary for the measurement port arrangement above ensures that the measurement port is arranged away from a section, in which a large flow velocity of the (air) flow through the inhalation therapy device negatively affects the measurement/detection of the pressure values inside the flow path.

According to a sixteenth aspect, the measurement port is arranged in an area of the flow path, in which the share of the dynamic pressure with respect to the total pressure measured at the measurement port is between 10% and 0.0001%.

It is, in this connection, preferred to arrange the measurement port in an area of the flow path, in which the share of the dynamic pressure with respect to the total pressure measured at the measurement port is between 5% and 0.001%.

In line with the above, also the "share of the dynamic pressure with respect to the total pressure measured at the measurement port" is to be obtained via measurement during the sinus-shaped breathing pattern of two seconds of inhalation time and exhalation time, respectively, and 500ml of tidal volume.

According to a seventeenth aspect, the measurement port comprises the measurement surface. The measurement port is arranged in an area of the flow path in which, normal to the measurement surface at the position of the measurement port with a distance of 1mm to the measurement port, a flow velocity is in a range of O.Olm/s to 4m/s, preferably of 0.1m/s to 2m/s.

In other words, when the flow velocity is to be measured above the measurement surface of the measurement port in a distance of 1 mm away from it, the flow velocity shall be in the above-recited range(s).

Also here, the flow velocities are to be measured via a measurement during the sinus-shaped breathing pattern of two seconds of inhalation time and exhalation time, respectively, and 500ml of tidal volume.

In both, the sixteenth and seventeenth aspect, the particularly small share of existent dynamic pressure (compared to the total pressure measured at the measurement port) and/or the flow velocity ranges as recited above ensure that a permanent contamination due to, for example, liquids, germs, and/or sputum can be prohibited, and the quality of detecting/measuring the flow parameter inside the flow path, preferably the pressure inside the flow path during inhalation, preferably and exhalation, can be achieved.

### Brief Description of The Drawings

Hereinafter, non-limiting examples of the disclosure are explained with reference to the drawings, in which:
Fig. 1 shows an embodiment of the inhalation therapy device according to the present disclosure in an isometric view.
Fig. 2 shows a schematic cross-sectional view along the flow path of the inhalation therapy device of Fig. 1.

### Detailed Description of Preferred Embodiments

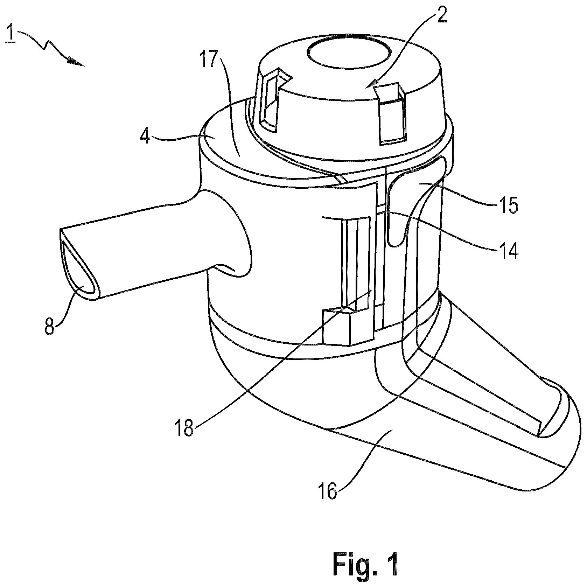

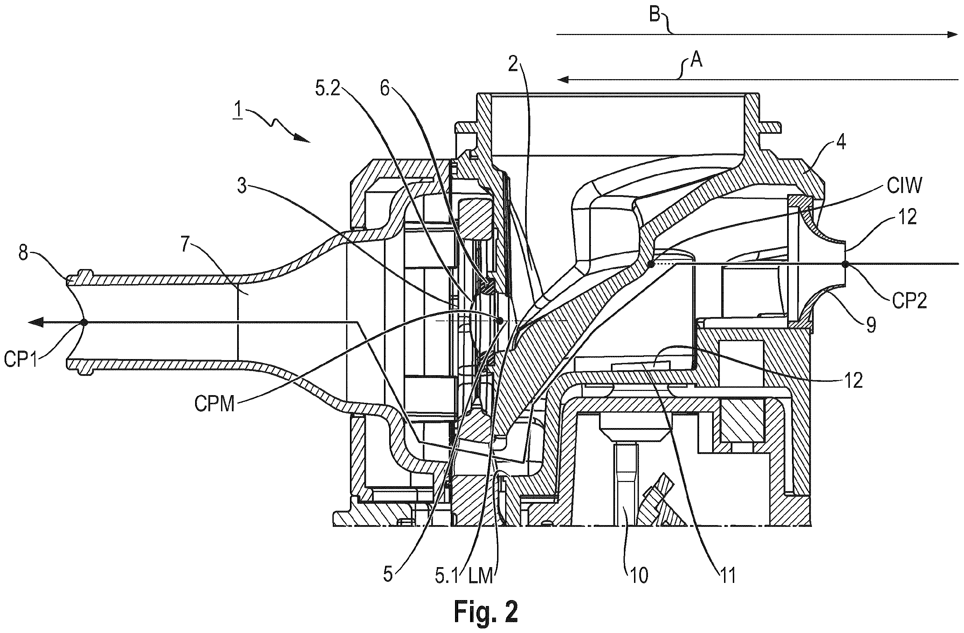

Currently preferred embodiments of the present disclosure will now be described with reference to the accompanying drawings. The preferred embodiments refer to an inhalation therapy device 1. In the following a preferred embodiment of the inhalation therapy device of the present disclosure will be described with reference to Figs. 1 and 2.

It is to be understood that such inhalation therapy devices 1 are oftentimes also called "aerosol delivery devices" or "nebulisers", for example, for a therapeutic use via a user's respiratory system.

Fig. 1 shows an exemplary isometric view of the inhalation therapy device 1 according to a currently preferred embodiment of the present disclosure.

It is derivable therefrom, that the overall shape of the inhalation therapy device 1 is split into two main parts. The inhalation therapy device 1 comprises a holding portion 16, which may also be called "controller portion" and an aerosolization portion 17. Yet, the present disclosure is not limited to such a "split" configuration: It may also be possible to form the holding portion 16 and the aerosolization portion 17 as an integral element. While the holding portion 16 as illustrated in Fig. 1 aims to enable that the inhalation therapy device 1 can be held by a user during (aerosol) therapy, the aerosolization unit 17 itself is provided to aerosolise or nebulise a fluid or liquid (i.e., a medicament) via a membrane unit (which is described in more detail with reference to Fig. 2 below).

The holding portion 16 may comprise the most relevant, preferably all, electronic features necessary to operate the inhalation therapy device 1 such that the aerosolization unit 17 can be configured as a throwaway product or a product, which is to be used for a certain amount of time, such as a certain number of therapies, weeks, or months. At the same time, the holding portion 16 comprising all relevant electric and sensory elements for operating the inhalation therapy device 1 and monitoring the therapy process may be construed as a long-lasting element, which has lower requirements regarding cleaning and/or disinfection compared to the aerosolization unit 17.

To enable a decent cleanability of the aerosolization unit 17 and an easy access of the membrane unit 3 arranged inside the aerosolization unit 17, the exemplary embodiment according to Fig. 1 provides a latch 18. The latch 18 enables access to the interior of the inhalation therapy device 1, especially to the membrane unit 3 inside the aerosolization unit 17 of the inhalation therapy device 1. Yet, providing the latch 18 is not compulsory. It is also possible to provide aerosolization units 17 that cannot be opened by a user, operator, hospital staff, or the patient itself. This is, for example, achievable by forming an aerosolization unit 17, in which the membrane unit 3 is arranged in a fixed position and cannot be accessed/replaced.

In the technical field given, such membrane units 3 are also known as "head units". Similarly, the aerosolization unit 17 is known as a "nebset", which reflects an abbreviation of nebulisation set.

The preferred embodiment illustrated in Fig. 1 is an inhalation therapy device 1 through which a user inhales and exhales during the therapy. That is, the inhalation therapy device 1 is brought into contact with the user's mouth, preferably mouth and nose, for example, via a mask, such that the regular inhalation and exhalation of the user is performed through the inhalation therapy device 1. That is, the inhalation therapy device 1, specifically its first opening 8 (which will be described in more detail below) shall not be removed from the user's mouth (and nose) during the aerosol therapy.

How the aerosol is generated and how the actual therapy is to be performed inside the inhalation therapy device 1 will now be described in more detail with reference to Fig. 2.

Fig. 2 shows a cross-sectional view along a flow path 7 through the inhalation therapy device 1 and, in this connection, focusses on the (upper) aerosolization unit 17 of the inhalation therapy device 1 of Fig. 1. Accordingly, merely the uppermost section of the holding portion 16, when the inhalation therapy device 1 is held in an ideal position by a user during therapy in a sitting position of the user and an upright position of the user's head, is illustrated in Fig. 2.

The circumference of the inhalation therapy device 1, especially of its aerosolization unit 17, is defined by a housing body 4.

Here and in the following, it shall once again be noted that Figure 2 shows the orientation of the inhalation therapy device when it is held by a patient in an ideal position during therapy in the sitting position of the patient and an upright position of the patient's head.

In this scenario, a reservoir 2 for holding a liquid or fluid (i.e., the medicament) is provided at an upper section of the housing body 4. It is apparent from Fig. 1 that a lid is provided on the upper section of the reservoir 2 for ensuring that the fluid or liquid in the reservoir 2 is not spilled during therapy. Yet, in Fig. 2, said lid is not illustrated for easier orientation.

Additionally, it is derivable from Fig. 2 that also a membrane unit 3 is provided in the housing body 4.

To produce the aerosol for therapy, the membrane unit 3 comprises a membrane 5 and an actuator 6. In the cross-sectional view of Fig. 2, the membrane 5 is arranged in an upright position, i.e., in a vertical position, in the housing body 4, such that the membrane 5 can encounter the fluid or liquid being held in the reservoir 2. In other words, the reservoir 2 and the membrane 5 are disposed adjacent to each other, such that the fluid or liquid can be supplied to a first side 5.1 of the membrane 5, when a fluid or liquid is held in the reservoir 2. Doing so enables that the fluid or liquid is automatically, i.e., gravitationally, transported to the membrane 5 of the membrane unit 3 for producing aerosol for the user's therapy. This is achieved by arranging the membrane 5 at a lowermost position of the reservoir 2 when the inhalation therapy device 1 is held by a user during therapy (see Fig. 2).

The membrane 5 comprises a plurality of (non-illustrated) apertures and the actuator 6 is coupled to the membrane 5, such that it can vibrate the same. By doing so, the liquid passes through the apertures, and aerosol is generated at a second side 5.2 of the membrane 5 opposite of the first side 5.1 of the membrane 5. That is, an aerosol bolus, which may also be understood as an "aerosol plume", is generated in the chamber on the in Fig. 2 left-hand side of the membrane unit 3 inside the housing body.

To entrain and deliver the generated aerosol at the second side 5.2 of the membrane 5 inside the housing body 4 of the inhalation therapy device 1 to the user's respiratory tract, the inhalation therapy device 1 comprises a flow path 7.

The flow path 7 comprises a first opening 8 to the outside of the housing body 4 at one end thereof and a second opening 9 to the outside of the housing body 4 at another end. In the illustrated embodiment of Fig. 2, the first opening 8 and the second opening 9 are arranged at opposing sides of the housing body 4. Yet, said arrangement is not binding and it may also be possible that, for example, the position of the second opening 9 is arranged offset from a longitudinal axis through the first opening 8 of the flow path 7.

As mentioned earlier, it is the first opening 8 that is to be brought into contact, may it be directly or indirectly, for example, via a mouthpiece or a mask, with the user's respiratory tract via his or her mouth, and optionally his or her nose, to enable inhalation and exhalation through the inhalation therapy device 1 during the aerosol therapy.

Vice versa, the second opening 9 allows air to enter the flow path 7 and to be expelled therefrom. Hence, the flow path 7 and its first opening 8 and second opening 9 to the outside of the housing body 4 enable that a flow is generatable in the first flow direction from the second opening 9 to the first opening 8 in the flow path 7 upon inhalation of the user at the first opening 8. This flow may be understood as an inhalation flow, wherein the entire flow volume along the first flow direction A to the first opening 8 passes through the second opening 9 into the flow path 7 past a measurement port 11 (which will be described in more detail below) and the membrane unit 3, and merges with the aerosol at the second side 5.2 of the membrane 5 before it gets expelled from the inhalation therapy device 1 into the user's body at the first opening 8 of the flow path 7.

In the embodiment of Fig. 2, also the entire flow volume along the second flow direction B, i.e., the direction of the exhalation flow from the first opening 8 to the second opening 9, passes through the second opening 9 upon exhalation of the user at the first opening into the flow path 7.

Fig. 2 illustrates the first flow direction A from the second opening 9 to the first opening 8, i.e., the direction of the inhalation flow, and the second flow direction B from the first opening 8 to the second opening 9 in the flow path 7, i.e, the direction of the exhalation flow.

Regarding the shape of the flow path 7, it is apparent from Fig. 2 that the flow path 7 is shaped to prohibit a direct (un-redirected) streaming of air from the second opening 9 to the first opening 8 of the flow path 7 and vice versa. In fact, a centre point CP1 of the cross-section of the first opening 8, a centre point CPM of the cross-section of the membrane 5, and a centre point CP2 of the cross-section of the second opening 9 do not align with each other in one single axis. In other words, at least one of these three centre points CP1, CPM, CP2 of the first opening 8 and second opening 9 and the membrane 5 is arranged offset, thereby provoking a redirection of the air through the inhalation therapy device 1, and thereby omitting a "pipe shaped" flow path structure.

The membrane unit 3 is disposed in the flow path 7, and thereby in said "flow" generatable by the user's breathing, especially his or her inhalation and exhalation, in such a manner that it can be surrounded by the flow through the flow path 7. As illustrated, the membrane unit 3 is arranged in an axially and circumferentially centred position of the flow path 7, such that a sheathing enveloping flow can pass the entire of the membrane unit 3 inside the flow path 7 during inhalation and exhalation of the user by breathing through the inhalation therapy device 1 during therapy.

The curved arrow of Fig. 2 extending from the second opening 9 to the first opening 8 represents an exemplary flow of the air along the first flow direction A (i.e., an inhalation (air) flow). It should, however, be understood that this exemplary flow path 7 is not limited to the flow passing underneath the membrane unit 3 passed a lowermost position LM of the flow path 7. This is merely one example of a flow through the cross-sectional illustration of the flow path 7. In reality, the flow passes the membrane unit 3 and the illustrated reservoir 2, for example, also on the sides of the membrane unit 3, as it is arranged in such a manner that an envelope flow is generated about the circumference of the membrane 5.

It is the air flow that is redirected to be guided around the membrane unit 3 for entraining and delivering the generated aerosol upon inhalation. At the same time, the lowermost position LM of the flow path 7 is arranged underneath the membrane unit 3 in the exemplary embodiment of Fig. 2 and, thereby, acts as a "trap" for excessive liquids and/or sputum that accidently enters the flow path 7, especially during therapy.

The aerosol generated at the second side 5.2 of the membrane 5 can merge with the air guided from the second opening 9 through the flow path 7 along the first flow direction A in the area in front the second side 5.2 of the membrane 5 and can, accordingly, be "transported" to the first opening 8 for entraining and delivering the generated aerosol to the respiratory tract of the user.

This is the case, as the user's inhalation at the first opening 8 establishes the above-mentioned "flow" due to suction force to the air being inside the flow path 7 and air getting sucked into the flow path 7 via its second opening 9.

To trigger the actuator 6 and to evaluate the quality of the inhalation therapy, it is required to observe the way the inhalation and the exhalation through the inhalation therapy device 1 is performed.

Hitherto, the preferred embodiments of the present disclosure found an easy to manufacture and disinfect solution that reliably monitors the flow parameters inside the flow path 7 and, thereby, improves the overall therapy success.

To do so, the pressure and negative pressure inside the flow path 7 upon inhalation and exhalation is measured as an exemplary form of a flow parameter at a position in the flow path 7 that will now be described in more detail.

The inhalation therapy device 1 comprises a sensor 10, which is configured to detect a pressure value inside the flow path 7. To do so, the sensor 10 comprises a measurement port 11, which is arranged inside the flow path 7. The measurement port 11 may also be understood as a "sensor port". The sensor 10 can detect the above-mentioned pressure values for evaluating at least the inhalation flow in the flow path 7 via said measurement port 11.

As illustrated in Fig. 2, the provision of a measurement port 11 allows a distant arrangement of the sensor 10 from the measurement port 11 inside the flow path 7 and a connection of the measurement port 11 and the sensor 10 via a hose, a molded pipe, or the like. Thus, the sensor 10 can, for example, be arranged in the holding portion 16, whereas the measurement port 11 is arranged in the flow path 7 through the housing body 4 of the aerosolization unit 17.

The pressure sensor 10 of the exemplary embodiment is a differential pressure sensor that determines a static pressure and compares the same to the environmental pressure, i.e., a reference pressure, which may also be understood as an ambient pressure.

The flowing pressure values, flow velocities, etc. particularly occur throughout the flow rates in a sinus-shaped breathing pattern of two seconds of inhalation time and two seconds of exhalation time and a tidal volume of 500ml. This is precisely defined in DIN ISO 27427. Hence, to allow comparisons to conventional solutions, said sinus-shaped breathing pattern of two seconds of inhalation time and two seconds of exhalation time and a tidal volume of 500ml shall be applied.

In the exemplary embodiment of Fig. 2, not only the measurement port 11, but also the second opening 9 of the flow path 7 is arranged upstream of the membrane unit 3, when seen in the first flow direction A. It is, in this connection, apparent from Fig. 2 that the second opening 9 is arranged at an upper half of the housing body 4.

It is furthermore apparent from Fig. 2 that the measurement port 11 in the exemplary embodiment of Fig. 2 is disposed substantially in the middle between the measurement port 11 and the second opening 9 in the flow path 7, when seen along the first flow direction A.

However, this arrangement is not particularly binding, and it may also be possible to arrange the measurement port 11 closer to the second opening 9 than to the membrane unit 3 in the flow path 7 or even to arrange the measurement port 11 closer to the membrane unit 3 than to the second opening 9 in the flow path 7.

The second opening 9 is oriented and shaped in such a manner that the inhalation flow is at least partially guided towards an inner wall of the flow path 7, wherein the flow contacts the wall of the flow path 7 at point CIW and a region around it and gets, thereby, redirected.

Irrespective of the measurement port's arrangement along the first flow direction A upstream of the membrane unit 3, it is apparent from Fig. 2 that the flow path 7 comprises a protruding portion 12 that protrudes from the inner wall of the flow path 7, and the measurement port 11 is arranged on the top section of the protruding portion 12. In the exemplary embodiment of Fig. 2, said protruding portion 12 protrudes into the flow path 7 by 0.75 mm. Yet, this is merely an example, and a protrusion height of 1mm, 1.3mm, or even 1.5mm away from the wall of the flow path 7 may also be possible.

Based on these structures, the measurement port 11 in the exemplary embodiment is arranged at a position that a) differs from said lowermost position LM of the flow path 7, and/or b) is sufficiently far away from any wall portion of the flow path 7 via the protruding portion 12, and/or c) is sufficiently far away from the main flow (path) of the air through the flow path 7, especially from the contact area of the inhalation flow at one of the walls (i.e., point CIW) . In this connection, it is apparent from Figure 2 that the measurement shall preferably be arranged at least 5mm way from the area at point CIW, where the inhalation flow contacts the inner wall of the flow path 7. This is highlighted by the dashed line in Fig. 2.

Requirements a), b), and/or c) are beneficial, as it can therewith be ensured that the dynamic pressure caused by air entering the flow path 7 and passing the measurement port 11 is not provoking a major influence on the detected measurement values. At the same time, a deposition of contaminating elements, such as sputum, liquids, or germs on the measurement port 11 can effectively be prevented.

Alternatively or additionally, the advantageous arrangement of the measurement port 11 may be defined based on areas, in which the share of the dynamic pressure with respect to the total pressure measurable at said measurement port 11 is between 10% and 0.001%, preferably between 5% and 0.001%.

That is, the measurement port 11 is arranged sufficiently far away from an area, in which the dynamic pressure is large and could thereby, deteriorate the measurement quality of the pressure detection inside the flow path 7 via sensor 10 and its measurement port 11.

Alternatively or additionally, the advantageous arrangement of the measurement port 11 may be defined based on areas, in which the ratio of the relative static pressure at the measurement port 11 to the relative static pressure at the first opening 8 is between 80% and 120%, preferably between 90% and 110%, more preferably between 95% and 105%.

Equally, the measurement port 11 may be arranged in an area of the flow path 7, in which the dynamic pressure is between 0.0001% and 40% of the maximum dynamic pressure inside the flow path.

As illustrated in Fig. 2, the measurement port 11 comprises a measurement surface. Based thereon, the advantageous arrangement of the measurement port 11 may be defined based on areas, in which, normal to the measurement surface at the position of the measurement port 11 with a distance of 1mm to the measurement port 11, a flow velocity is in a range of O.Olm/s to 4m/s, preferably of 0.1m/s to 2m/s.

It is to be understood that, irrespective of the question whether suitable areas for arranging the measurement port 11 are defined via the above-discussed boundaries of the flow velocity, dynamic pressures (compared to the total pressure), relative static pressures, or the like, all of said values are to be determined during the application of a sinus-shaped breathing pattern of two seconds of inhalation time and exhalation time, respectively, and 500ml of tidal volume to the inhalation therapy device. Particularly the flow velocities are to be measured at the peak flow of said sinus-shaped breathing pattern of two seconds of inhalation time and exhalation time, respectively, and 500ml of tidal volume.

The configuration as illustrated in Figures 1 and 2 fulfils all the constraints discussed above. It can therewith be ensured that the dynamic pressure caused by air entering the flow path 7 and passing the measurement port 11 is not provoking a major influence on the detected measurement values. At the same time, a deposition of contaminating elements, such as sputum, liquids, or germs on the measurement port 11 can effectively be prevented.

As illustrated In Figure 2, the measurement surface of the measurement port may, for example, also be inclined to the longitudinal axis of the first opening 8. This may equally be understood as an inclination in relation to the horizontal when, as illustrated in Fig. 2, the inhalation therapy device 1 is held in an ideal position by the user during therapy in sitting position of the user.

The second opening 9 may be shaped in such a manner that it can be considered as a flow restrictor. To achieve the flow resistance, a geometrical form is to be established that provokes a higher flow resistance in the inhalation flow direction than in the exhalation flow direction. Typical relationships of the inhalation flow resistance to the exhalation flow resistance established by the second opening's shape are 125%, 130%, 150%, 175%, 190% or even 300%, 400%, or 500%. In a most preferred embodiment, the relationship of the inhalation flow resistance to the exhalation flow resistance established by the second opening's shape is 190%. This enables to advantageously prolong the inhalation time, while the exhalation time can be shortened.

The actual shape of the second opening 9, which is responsible to consider it as the above-mentioned flow restrictor, will now be described in more detail with reference to Fig. 2.

To do so, the flow path's second opening 9 is shaped as a fixed geometry passive valve. The second opening 9 may be formed as an integral part of the housing body 4 or as a separate structural element being connected to the housing body 4 and forming the outlet of the flow path 7 through said housing body 4 for delivering the generated aerosol to the user during therapy.

It can be taken from Fig. 2 that the cross-sectional shape of the second opening 9 continuously decreases along the second flow direction B. This continuously decreasing cross-sectional shape of the second opening 9 along the second flow direction B enables that the flow resistance in the second flow direction B can be made lower than in the first flow direction A without the need of any active elements such as actuated valves or the like.

Yet, it is not necessarily required to establish said relationships of the inhalation flow resistance to the exhalation flow resistance with one single second opening 9 in the housing body 4 only. The shape of the second opening 9 provokes that air that enters the flow path 7 via the second opening 9 upon inhalation is confronted with a narrowed cross-sectional shape and an arres, i.e., a sharp edge at the outermost position of the second opening 9. This shape provokes a flow separation at the outermost position of the second opening 9 and thereby leads to an increased flow resistance upon inhalation due to the urge of increasing the velocity. At the same time, the decreasing cross-sectional shape along the second flow direction B provokes a reduced exhalation resistance due to the lack of sharp edges and in the following avoids flow separation along the flow along the second flow direction B.

This leads to a facilitated exhalation through the inhalation therapy device 1 and thereby, a more pleasant feeling for the user, as the exhaustion of air does not have to be performed against a large resistance. Accordingly, the inhalation time can be prolonged, and the exhalation time can be shortened, resulting in a high duty cycle, and, as a result, a short overall therapy time.

It may equally be possible that the relationship of the inhalation flow resistance to the exhalation flow resistance is achieved by a plurality of sub-openings that together form the above-mentioned flow resistances of the second opening 9.

In another embodiment, it may also be possible that another, third opening 14 to the outside of the housing body 4 is provided in the flow path 7 between said first opening 8 and the second opening 9.

This is, for example, illustrated in Fig 1. The third opening 14 is arranged upstream of the membrane unit 3 when seen in the first flow direction A. Further, the third opening 14 is provided with a check valve 15, which is configured to restrict a flow through the third opening 14 in the first flow direction A. That is, the check valve 15 prohibits any flow through the third opening 14 of the flow path 7 upon inhalation. However, the check valve 15 is also configured to enable a flow through the third opening 14 in the second flow direction B from the first opening 8 to the second opening 9 for enabling at least a partial, preferably substantially a full expel of air through the third opening 14.

This configuration allows to achieve the above-desired inhalation flow resistance and, at the same time, allows to reduce the exhalation flow resistance even further, as the check valve 15 facilitates the expel of air via the check valve 15 and the third opening 14 of the flow path 7.

Irrespective of the presence of a third opening 14 or not, the inhalation therapy device 1 may, in a further, not illustrated embodiment, comprise an air filter device, which is reversibly attachable and detachable to the housing body 4. Said air filter device is configured to filter harmful substances from the exhalation flow. The exhalation flow may contain substances and/or aerosols, which may contain materials or germs that may be harmful for the environment. Provided that the third opening 14 is present, the air filter device is shaped to cover said third opening 14 and its check valve 15, such that the flow in the second flow direction B primarily passes through the air filter device to the outside of the housing body 4 upon exhalation. Vice versa, if the third opening 14 is not present and the flow path 7 extends between the first opening 8 and the second opening 9 only, the air filter device is shaped to cover the second opening 9, which is, besides the first opening 8, the only opening of the flow path 7 to the outside of the housing body 4, such that the entire flow volume along the first flow direction A passes through the air filter device upon inhalation, and, upon exhalation, the entire flow volume along the second flow direction B passes through the air filter device.

### Reference Signs List

- 1: inhalation therapy device
- 2: reservoir
- 3: membrane unit
- 4: housing body
- 5: membrane
- 5.1: first side of the membrane
- 5.2: second side of the membrane
- 6: actuator
- 7: flow path
- 8: first opening
- 9: second opening
- 10: sensor
- 11: measurement port
- 12: protruding portion
- 14: third opening
- 15: check valve
- 16: holding portion
- 17: aerosolization portion
- 18: latch
- A: first flow direction
- B: second flow direction
- CP1: centre point (of the cross-section of the first opening)
- CPM: centre point (of the cross-section of the membrane)
- CP2: centre point (of the cross-section of the second opening)
- LM: lowermost position

## Claims

1. Inhalation therapy device (1), comprising:
a housing body (4),
a reservoir (2) for holding a liquid,
a membrane unit (3) comprising:
a membrane (5) disposed in the housing body (4) and having a plurality of apertures, wherein the membrane (5) is disposed so that, when liquid is held in the reservoir (2), the liquid is supplied to a first side of the membrane (5.1), and
an actuator (6) coupled to the membrane (5) for vibrating the membrane (6), whereby the liquid passes through the apertures and an aerosol is generated at a second side (5.2) of the membrane (5) opposite to the first side (5.1) of the membrane (5),
wherein the inhalation therapy device (1) further comprises a flow path (7) being defined in the housing body (4) and having a first opening (8) to the outside of the housing body (4) at one end and a second opening (9) to the outside of the housing body (4) at another end, so that at least an inhalation flow (A) from the second opening (9) to the first opening (8) is generatable in the flow path (7) upon inhalation of a patient at the first opening (8) for entraining and delivering the generated aerosol, and
wherein the membrane unit (3) is disposed in the flow path (7) between the first opening (8) and the second opening (9),
**characterized by**
a sensor (10) comprising a measurement port (11), the sensor (10) being configured to detect a flow parameter of a flow in the flow path (7) via the measurement port (11), and
the measurement port (11) being disposed upstream of the membrane unit (3) in the flow path (7), when seen in the direction of the inhalation flow (A).

2. Inhalation therapy device (1) according to claim 1, wherein the sensor (10) is configured to at least detect, preferably measure, a pressure of the inhalation flow (A) in the flow path (7), preferably and a pressure of an exhalation flow (B) from the first opening (8) to the second opening (9) in the flow path (7) upon exhalation of the user at the first opening (8) into the flow path (7).

3. Inhalation therapy device (1) according to any of the preceding claims, wherein the membrane unit (3) is arranged in such a manner in the flow path (7) that it is at least partially surrounded by the flow in the flow path (7).

4. Inhalation therapy device (1) according to any of the preceding claims, wherein the flow path (7) is shaped in such a manner that the entire inhalation flow passes through the flow path (7) upon inhalation of the user.

5. Inhalation therapy device (1) according to any of the preceding claims, wherein the measurement port (11) is disposed substantially in the middle between the membrane unit (3) and the second opening (9) in the flow path (7), or is disposed closer to the second opening (9) than to the membrane unit (3) in the flow path (7), or is disposed closer to the membrane unit (3) than to the second opening (9) in the flow path (7).

6. Inhalation therapy device (1) according to any of the preceding claims, wherein the measurement port (11) comprises a measurement surface,
the measurement surface being inclined to the longitudinal axis of the first opening (8), or
the measurement surface being an inclined plane in relation to the horizontal, when the inhalation therapy device (1) is held by a user during therapy in a sitting position of the user and a head of the user is in an upright position.

7. Inhalation therapy device (1) according to any of the preceding claims, wherein the measurement port (11) is arranged at a position other than a lowermost position (LM) of the flow path (7), when the inhalation therapy device (1) is held by the user during therapy in the sitting position of the user and the head of the user is in the upright position.

8. Inhalation therapy device (1) according to any of the preceding claims, wherein a protruding portion (12) protruding into the flow path (7) is arranged inside the flow path (7), and wherein the measurement port (11) is arranged on the protruding portion (12).

9. Inhalation therapy device (1) according to claim 8, wherein the protruding portion (12) protrudes into the flow path (7) by at least 0.5mm, preferably by at least 1.5mm.

10. Inhalation therapy device (1) according to any of the preceding claims, wherein the flow path (7) is shaped in such a manner that a centre point (CP1) of the cross-section of the first opening (8), a centre point (CPM) of the cross-section of the membrane (5), and a centre point (CP2) of the cross-section of the second opening (9) do not align with each other.

11. Inhalation therapy device (1) according to any of the preceding claims, wherein the second opening (9) is shaped in such a manner that the inhalation flow (A) is at least partially guided towards an inner wall of the flow path (7).

12. Inhalation therapy device (1) according to claim 11, wherein the measurement port (11) is arranged away, preferably at least 5mm away, from an area (CIW), where the inhalation flow contacts the inner wall of the flow path (7).

13. Inhalation therapy device (1) according to any of the preceding claims, wherein the measurement port (11) is arranged offset from a vertical projection of the cross-section of the second opening (9).

14. Inhalation therapy device (1) according to any of the preceding claims, wherein the measurement port (11) is arranged in an area of the flow path (7), in which a dynamic pressure is between 0.0001% and 40%, preferably between 0.0005% and 20%, more preferably between 0.001% and 5%, most preferably between 0.002% and 2%, of the maximum dynamic pressure inside the flow path (7), the dynamic pressure being measured during a sinus-shaped breathing pattern of two seconds of inhalation time and exhalation time, respectively, and 500ml of tidal volume.

15. Inhalation therapy device (1) according to any of the preceding claims, wherein the measurement port (11) is arranged in an area of the flow path (7), in which the ratio of the relative static pressure at the measurement port (11) to the relative static pressure at the first opening (8) is between 80% and 120%, preferably between 90% and 110%, more preferably between 95% and 105%,
the relative static pressures being measured during a sinus-shaped breathing pattern of two seconds of inhalation time and exhalation time, respectively, and 500ml of tidal volume, and
wherein the relative static pressure reflects a difference between a local static pressure and an ambient pressure.

16. Inhalation therapy device (1) according to any of the preceding claims, wherein the measurement port (11) is arranged in an area of the flow path (7), in which the share of the dynamic pressure with respect to the total pressure measured at the measurement port (11) during a sinus-shaped breathing pattern of two seconds of inhalation time and exhalation time, respectively, and 500ml of tidal volume is between 10% and 0.0001%, preferably between 5% and 0.001%.

17. Inhalation therapy device (1) according to any of the preceding claims, wherein
the measurement port (11) comprises the measurement surface, and
the measurement port (11) is arranged in an area of the flow path (7), in which, normal to the measurement surface at the position of the measurement port (11) with a distance of 1mm to the measurement port (11), a flow velocity is in a range of O.Olm/s to 4m/s, preferably of 0.1m/s to 2m/s, the velocity being measured at the peak flow of a sinus-shaped breathing pattern of two seconds of inhalation time and exhalation time, respectively, and 500ml of tidal volume.
